# EUROPÄISCHE PATENTSCHRIFT

(11) **EP 2 482 729 B1**
(45) Veröffentlichungstag und Bekanntmachung des Hinweises auf die Patenterteilung: **16.07.2014**
(21) Anmeldenummer: 10763349.7
(22) Anmeldetag: 28.09.2010
(51) Int. Cl.: A61B 6/00, H05G 1/70, A61B 6/03

(54) **MULTI-SOURCE CT-SYSTEM**
MULTI-SOURCE CT SYSTEM
SYSTEME DE TOMOGRAPHIE PAR ORDINATEUR A SOURCES MULTIPLES

(30) Priorität: 30.09.2009 DE 102009043221
(43) Veröffentlichungstag der Anmeldung: 08.08.2012
(73) Patentinhaber: Siemens Aktiengesellschaft, 80333 München (DE)
(72) Erfinder: FRITZLER, Sven, 64747 Breuberg-Sandbach (DE); MATSCHULLA, Jan, 02791 Oderwitz (DE)
(86) Internationale Anmeldenummer: PCT/EP2010/064365
(87) Internationale Veröffentlichungsnummer: WO 2011/039189

(56) Entgegenhaltungen:
- WO-A1-2004/080310
- WO-A1-2008/122970
- WO-A2-2006/090323
- DE-A1- 10 346 682
- US-B1- 7 453 976

## Beschreibung

Die Erfindung betrifft ein Multi-Source CT-System gemäß Oberbegriff des Anspruches 1.

Ein derartiges Multi-Source CT-System, das zur Erzeugung von Röntgenaufnahmen eines Untersuchungsobjektes dient, ist aus der US 7,453,976 B1 bekannt. Das bekannte Multi-Source CT-System umfasst wenigstens eine erste und eine zweite um eine Rotationsachse drehbar gelagerte und jeweils ein Röntgenstrahlenbündel aussendende Röntgenstrahlenquelle, wobei sich die erste (2) und zweite (4) Röntgenstrahlenquelle technisch voneinander unterscheiden, sowie wenigstens einen von den Röntgenstrahlenbündeln getroffenen Röntgenstrahlendetektor.

Ähnliche Multi-Source CT-Systeme sind jeweils für sich auch aus der WO 2006/090323 A2 und der WO 2004/080310 A1 sowie der WO 2008/122970 A1 bekannt.

Eine wesentliche Zukunftsanforderung an Computertomographie-(CT-) Scanner sind immer höhere Rotationsfrequenzen der Gantry. Eine schnellere Rotation der Gantry um den Patienten verkürzt die Aufnahmezeit, was wiederum in einer höheren zeitlichen Auflösung der Aufnahmen resultiert. Dies ist insbesondere in der Kardiologie von großer Bedeutung, da hier am schlagenden Herzen eine hohe zeitliche Auflösung auch die Ortsauflösung beeinflusst und damit die Möglichkeit Verkalkungen der Arterien schon in einem frühen Stadium zu detektieren. Bei höheren Rotationsfrequenzen muss allerdings auch die mAs-Anforderung zum Erreichen einer bestimmten Bildqualität erfüllt werden. Damit müssen immer höhere Strahlungsleistungen gefahren werden, so dass diese Strahler speziell auf die geforderte Kurzzeitbelastung von typischerweise 1 s ausgelegt werden müssen.

Eine Erhöhung der Rotationsfrequenz erhöht jedoch im Gegenzug durch die erhöhten Fliehkräfte auch die Belastung auf die Komponenten der Gantry, so dass derzeit die Rotationsfrequenz auf 3 bis 4 Hz limitiert ist. Um dennoch die Zahl der pro Zeiteinheit gewonnenen Projektionen zu erhöhen, ist die Verwendung von Zwei- oder Mehrstrahler CT-Systemen bekannt, bei denen zwei oder mehr Röntgenstrahler-Detektor-Einheiten winkelversetzt in einem Gantrygehäuse angeordnet sind.

Weiterhin besteht eine bekannte Methode zur besseren Unterscheidung unterschiedlicher Gewebearten darin, den Untersuchungsbereich vorzugsweise gleichzeitig mit unterschiedlich spektralverteilter Röntgenstrahlung, d.h. mit unterschiedlicher maximalen Röntgenenergie oder unterschiedlichem spektralem Schwerpunkt aufzunehmen.

Aus der DE 10 2007 024 158 A1 ist ein Dual-Source CT-System zur Untersuchung eines Patienten bekannt, das ein Gantrygehäuse aufweist, in dem winkelversetzt zwei Röntgenröhren mit gegenüberliegenden Detektorsystemen angeordnet sind, die zur Abtastung des Patienten um eine Systemachse rotieren, während der Patient durch die steuerbare Patientenliege entlang der Systemachse durch den Messbereich des CT-Systems verschoben wird. Dabei werden bei einer Dual-Energy CT-Untersuchung die beiden gleichartigen Röntgenröhren mit unterschiedlichen Beschleunigungsspannungen betrieben, so dass sich die beiden verwendeten Röntgenspektren stark unterscheiden und auch unterschiedliche Absorptionswerte in den zugeordneten Detektoren bei der Durchstrahlung des Patienten liefern.

Neben den Kurzzeit-Scans mit maximal einigen wenigen Sekunden Dauer sollen die CT-Systeme aber auch für Ganzkörper-Scans und Perfusionsmessungen genutzt werden. Bei diesen muss der Röntgenstrahler wiederum auf Aufnahmezeiten von bis zu einer Minute ausgelegt werden. Da die jeweiligen Anforderungen technologisch nicht mit einer Maßnahme abgedeckt werden können, wird der Röntgenstrahler vor extreme Belastungen gestellt, was sich insbesondere auf dessen Lebensdauer niederschlägt.

Aufgabe der vorliegenden Erfindung ist es daher, ein Multi-Source CT-System hinsichtlich der Anforderungen für unterschiedliche Aufnahmearten zu optimieren.

Diese Aufgabe wird durch ein Multi-Source CT-System mit den Merkmalen gemäß Patentanspruch 1 gelöst.

Die Grundidee der Erfindung besteht darin, bei einem Multi-Source CT-System mit wenigstens zwei Röntgenstrahlenquellen unterschiedliche Röntgenstrahlenquellen vorzusehen, die sich insbesondere hinsichtlich ihres technischen Aufbaus und der für eine bestimmte Aufnahmedauer maximal erzeugbaren Röntgenstrahlungsleistung deutlich unterscheiden. Dabei ist wenigstens eine Röntgenstrahlenquelle für eine erste und wenigstens eine zweite Röntgenstrahlenquelle für eine zweite Betriebsart des CT-Systems optimiert. Das erfindungsgemäße CT-System umfasst nur eine technisch sehr aufwendige Röntgenstrahlenquelle, die für eine kurze Aufnahmedauer eine extrem hohe Röntgenstrahlungsleistung erzeugen kann. Die zweite Röntgenstrahlenquelle ist dagegen auf Dauerleistung ausgelegt. Vorzugsweise wird für Ganzkörper- oder Perfusionsmessungen ausschließlich die zweite Röntgenstrahlenquelle verwendet.

Durch die Erfindung wird es möglich, bei einem Multi-Source CT-System mit mehreren Röntgenstrahlenquellen diese jeweils für ihre spezielle Verwendung zu optimieren. Dies führt zu einer Kostenreduktion des betreffenden Multi-Source CT-Systems. Da die verwendeten Röntgenstrahlenquellen zumindest überwiegend nur noch in der Betriebsart betrieben werden, für die sie optimiert wurden, erhöht sich dadurch auch deren Lebensdauer.

Gemäß der Erfindung unterscheiden sich die erste und zweite Röntgenstrahlenquelle zumindest hinsichtlich der von ihnen für eine bestimmte Aufnahmendauer maximal erzeugbaren Röntgenstrahlungsleistung um einen Faktor. Vorzugsweise liegt dieser Faktor im Bereich von 1,3 bis 3.

Bei einer bevorzugten Ausführungsform der Erfindung weist die erste Röntgenstrahlenquelle ein Magnetlager für den Anodenteller auf. Dadurch werden Rotationsfrequenzen der Drehanode von bis zu 1000 Hz ermöglicht. Weiterhin vorteilhaft weist die erste Röntgenstrahlenquelle eine mit dem Anodenteller wärmegekoppelte Stahlfläche zur Wärmeabfuhr auf. Die erste Röntgenstrahlenquelle ist dabei für Aufnahmezeiten von weniger als 5 s ausgelegt und optimiert.

Die zweite Röntgenstrahlenquelle des erfindungsgemäßen Multi-Source CT-Systems ist im Vergleich zu der ersten Röntgenstrahlenquelle von einfacherer Bauart. Insbesondere handelt es sich dabei um einen für Dauerleistungen auf Zeitstrecken von weit über 40 s ausgelegten Drehkolbenstrahler mit einer mittels eines Kugel- oder Gleitlagers gelagerten Drehanode.

Die Erfindung wird nachfolgend anhand eines Ausführungsbeispiels näher erläutert. Es zeigen:
- Figur 1: ein erfindungsgemäßes Multi-Source CT-System,
- Figur 2: die Röntgenstrahlungsleistung in Abhängigkeit der Betriebsdauer für unterschiedliche Röntgenstrahlenquellen.

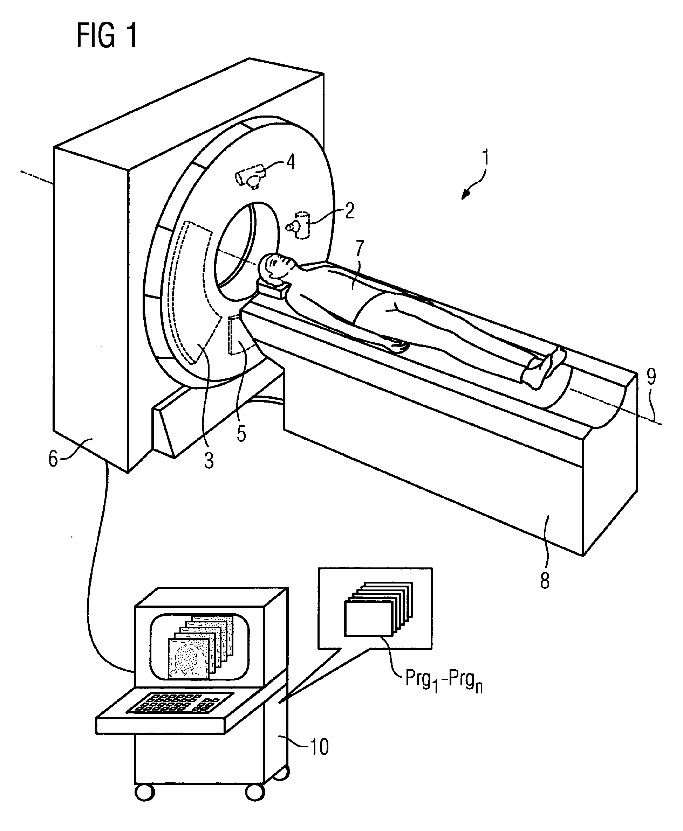

Ein Beispiel eines Dual-Source CT-Systems 1 gemäß der Erfindung ist in Figur 1 dargestellt. Dieses CT-System 1 weist ein Gantrygehäuse 6 auf, in dem winkelversetzt zwei Röntgenröhren 2 und 4 mit gegenüberliegenden Detektorsystemen 3 und 5 angeordnet sind, die zur Abtastung des Patienten 7 um eine Systemachse 9 rotieren, während der Patient 7 durch die steuerbare Patientenliege entlang der Systemachse 9 durch den Messbereich des CT-Systems verschoben wird. Zur Steuerung, Rekonstruktion und Durchführung des erfindungsgemäßen Verfahrens dient eine Steuer- und Recheneinheit 10, die in ihrem Speicher Computerprogramme Prg₁-Prgₙ beinhaltet, die im Betrieb die Steuerung und Rekonstruktion durchführen.

Zur besseren Unterscheidung unterschiedlicher Gewebearten im Untersuchungsbereich des Patienten 7 ist bei dem CT-System 1 ein Dual-Energy-Betrieb möglich. Hierzu muss die Röntgenabsorption zumindest bezüglich zweier unterschiedlicher Energiespektren gemessen werden. Dies geschieht mithilfe des Dual-Energy CT-Scanners 1, der es erlaubt, für mindestens einen axialen Schnitt durch den Patienten 7 zwei unabhängige Bilder zu rekonstruieren, die mit verschiedenen effektiven Röntgenspektren erzeugt wurden. Vorzugsweise erfolgt ein Simultan-Scan mit zwei verschiedenen Röhrenspannungen. Wichtig für die Bilderzeugung ist es, die unterschiedliche Absorptionswirkung auf die zwei unterschiedlichen Röntgenstrahlungsleistungen zu ermitteln.

In dem hier gezeigten Beispiel werden die beiden Röntgenröhren 2 und 4 mit unterschiedlichen Beschleunigungsspannungen betrieben, so dass sich die beiden verwendeten Röntgenspektren stark unterscheiden und auch unterschiedliche Absorptionswerte in den zugeordneten Detektoren bei der Durchstrahlung des Patienten 7 liefern.

Gemäß der Erfindung unterscheiden sich die beiden Röntgenröhren 2 und 4 in technischer Hinsicht. Von der Röntgenröhre 2 ist für eine kurze Aufnahmezeit im Bereich bis zu 5 s eine wesentlich höhere Röntgenstrahlungsleistung abgebbar als von der Röntgenröhre 4. Im Dual-Energy-Betrieb wird daher die Röntgenröhre 2 mit der höheren Beschleunigungsspannung betrieben. Im Unterschied dazu ist die Röntgenröhre 4 für eine Aufnahmezeit von 40 s und mehr ausgelegt. Allerdings ist dabei - auch kurzzeitig - keine so hohe Röntgenstrahlungsleistung wie mit der Röntgenröhre 2 erreichbar. Die Röntgenröhre 4 wird daher bei Dual-Energy-Scans mit der im Vergleich zu der Röntgenröhre 2 niedrigeren Beschleunigungsspannung betrieben. Weiterhin wird sie - vorzugsweise bei abgeschalteter Röntgenröhre 2 - für Ganzkörper-Scans und Perfusionsmessungen, die Aufnahmezeiten von 10 s und mehr erfordern, verwendet.

Bei der Röntgenröhre 2 handelt es sich um eine technisch aufwendige Röntgenröhre, bei der der Anodenteller mittels eines Magnetlagers gelagert ist und der auch über ein Magnetfeld angetrieben wird. Zur Wärmeableitung ist der Anodenteller mit einer Strahlfläche wärmegekoppelt. Weiterhin kann die Rotationsfrequenz des Anodentellers bis zu 1000 Hz betragen. Durch diesen Aufbau der Röntgenröhre 2 ist kurzzeitig eine extrem hohe Röntgenstrahlungsleistung erreichbar.

Im Unterschied hierzu handelt es sich bei der Röntgenröhre 4 um eine einfacher aufgebaute Drehkolbenröhre. Bei dieser ist zwar die maximal erreichbare Röntgenstrahlungsleistung wesentlich geringer als bei der Röntgenröhre 2 (beispielsweise nur 50% im Vergleich zur Röntgenröhre 2), dafür ist aber auch für Aufnahmezeiten von 1 min und mehr noch eine vergleichsweise hohe Röntgenstrahlungsleistung erreichbar.

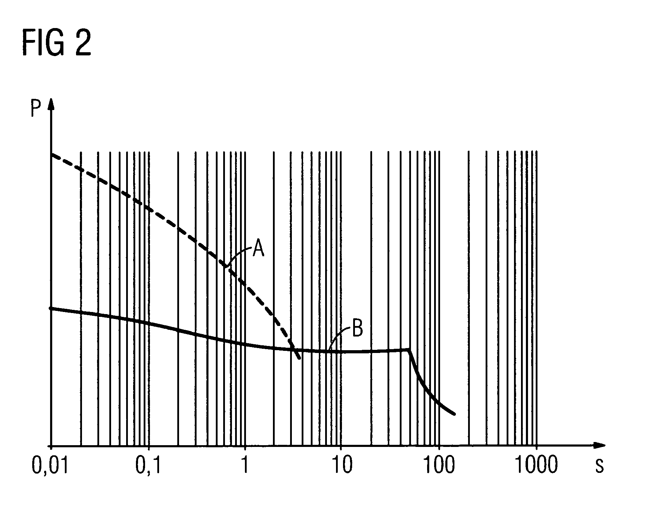

Den Zusammenhang zwischen der maximalen Röntgenstrahlungsleistung P in Abhängigkeit der Aufnahmezeit s veranschaulicht das Diagramm gemäß Figur 2.

Die Grenzlastkurve A der Röntgenröhre 2 zeigt eine deutlich höhere Röntgenstrahlungsleistung im Kurzzeitbereich bis zu einer Sekunde. Im Unterschied hierzu ermöglicht die Röntgenröhre 4 - veranschaulicht durch die Grenzlastkurve B - eine lange Dauerleistung bis zu 40 s, die deutlich oberhalb der Dauerleistung der Röntgenröhre 2 liegt.

Durch die Verwendung zweier technisch verschiedener Röntgenröhren 2 und 4 ist das erfindungsgemäße Dual-Source CT-System sowohl für Kurzzeit- als auch für Langzeitaufnahmen optimiert. Darüber hinaus ist auch ein Dual-Energy Betrieb möglich. Da die beiden Röntgenröhren 2 und 4 - im Vergleich zu herkömmlichen CT-Systemen - bei den damit möglichen, unterschiedlichen Aufnahmearten nicht am jeweiligen Limit betrieben werden müssen, wird die Lebensdauer der Röntgenröhren 2 und 4 deutlich erhöht und die Ausfallrate reduziert.

In einer besonderen Ausführungsform kann das "Dual Energy" Prinzip so erreicht werden, dass die Röntgenröhre 2 bei einer hohen Röhrenspannung betrieben wird, da hier für den gleichen Photonenfluss weniger Röhrenstrom benötigt wird. Die Röntgenröhre 4 wird hingegen mit einer geringeren Röhrenspannung und höherem Strom betrieben.

## Patentansprüche

1. Multi-Source CT-System (1) zur Erzeugung von Röntgenaufnahmen eines Untersuchungsobjektes, umfassend
- wenigstens eine erste (2) und eine zweite (4) um eine Rotationsachse drehbar gelagerte und jeweils ein Röntgenstrahlenbündel aussendende Röntgenstrahlenquelle, wobei sich die erste (2) und zweite (4) Röntgenstrahlenquelle technisch voneinander unterscheiden,
- wenigstens einen von den Röntgenstrahlenbündeln getroffenen Röntgenstrahlendetektor (3, 5),
**dadurch gekennzeichnet, dass**
die erste Röntgenstrahlenquelle (2) für einen Kurzzeitbetrieb mit einer hohen Röntgenstrahlungsleistung und die zweite Röntgenstrahlenquelle (4) für einen im Vergleich zu der ersten Röntgenstrahlenquelle (2) wesentlich längeren Betrieb mit niedrigerer Röntgenstrahlungsleistung optimiert ist.

2. Multi-Source CT-System (1) nach Anspruch 1, wobei die erste Röntgenstrahlenquelle (2) für eine erste Betriebsart des CT-Systems (1) und die zweite Röntgenstrahlenquelle (4) für eine zweite Betriebsart des CT-Systems optimiert ist.

3. Multi-Source CT-System (1) nach Anspruch 1 oder 2, wobei sich die erste (2) und zweite (4) Röntgenstrahlenquelle zumindest hinsichtlich der von ihnen für eine bestimmte Aufnahmedauer maximal erzeugbare Röntgenstrahlungsleistung um einen Faktor unterscheiden.

4. Multi-Source CT-System (1) nach Anspruch 3, wobei der Faktor größer 1,3 ist.

5. Multi-Source CT-System (1) nach einem der Ansprüche 1 bis 4, wobei die erste Röntgenstrahlenquelle (2) eine magnetisch gelagerte Drehanode aufweist.

6. Multi-Source CT-System (1) nach einem der Ansprüche 1 bis 5, wobei von der ersten Röntgenstrahlenquelle (2) für eine Betriebszeit von wenigstens 2 s eine Röntgenstrahlenleistung von mindestens 100 kW erzeugbar ist.

7. Multi-Source CT-System (1) nach einem der Ansprüche 1 bis 6, wobei die zweite Röntgenstrahlenquelle als Drehkolbenröhre ausgebildet ist.

8. Multi-Source CT-System (1) nach einem der Ansprüche 1 bis 7, wobei von der zweiten Röntgenstrahlenquelle (4) für eine Betriebszeit von mindestens 30 s eine Röntgenstrahlenleistung von mindestens 60 kW erzeugbar ist.

## Claims

1. Multi-source CT system (1) for the generation of X-ray images of an object under examination, comprising
- at least one first (2) and one second (4) X-ray radiation source, mounted in a rotatable manner about a rotational axis and in each case emitting a radiation beam, wherein the first (2) and second (4) X-ray radiation source differ from one another technically,
- at least one X-ray radiation detector (3, 5) struck by the radiation beams,
**characterised in that**
the first X-ray radiation source (2) is optimised for a short-term operation with a high X-ray radiation power and the second X-ray radiation source (4) is optimised for a significantly longer operation with lower X-ray radiation power in comparison to the first X-ray radiation source (2).

2. Multi-source CT system (1) according to claim 1, wherein the first X-ray radiation source (2) is optimised for a first mode of operation of the CT system (1) and the second X-ray radiation source (4) is optimised for a second mode of operation of the CT system.

3. Multi-source CT system (1) according to claim 1 or 2, wherein the first (2) and second (4) X-ray radiation source differ from each other by a factor at least in respect of the maximum X-ray radiation power they can generate for a particular recording duration.

4. Multi-source CT system (1) according to claim 3, wherein the factor is greater than 1.3.

5. Multi-source CT system (1) according to one of claims 1 to 4, wherein the first X-ray radiation source (2) has a magnetically mounted rotating anode.

6. Multi-source CT-System (1) according to one of claims 1 to 5, wherein an X-ray radiation power of at least 100 kW can be generated by the first X-ray source (2) for an operating duration of at least 2 s.

7. Multi-source CT system (1) according to one of claims 1 to 6, wherein the second X-ray radiation source is embodied as a rotary piston tube.

8. Multi-source CT-System (1) according to one of claims 1 to 7, wherein an X-ray radiation power of at least 60 kW can be generated by the second X-ray radiation source (4) for an operating duration of at least 30 s.

## Revendications

1. Système (1) de tomographie par ordinateur à sources multiples pour la production de radiographies d'un objet à analyser, comprenant
- au moins une première (2) et une deuxième (4) sources de rayons X montées tournantes autour d'un axe de rotation et émettant respectivement un faisceau de rayons X, la première (2) et la deuxième (4) sources de rayons X étant différentes techniquement l'une de l'autre,
- au moins un détecteur (3, 5) de rayons X sur le trajet du faisceau du rayon X,
**caractérisé en ce que**
la première source (2) de rayons X est optimisée pour un fonctionnement de courte durée à une grande puissance du rayonnement X et la deuxième source (4) de rayons X est optimisée à une puissance de rayonnement X plus basse pour un fonctionnement sensiblement plus long que la première source (2) de rayons X.

2. Système (1) de tomographie par ordinateur à sources multiples suivant la revendication 1, dans lequel la première source (2) de rayons X est optimisée pour un premier type de fonctionnement du système (1) de tomographie par ordinateur et la deuxième source (4) de rayons X pour un deuxième type de fonctionnement du système de tomographie par ordinateur.

3. Système (1) de tomographie par ordinateur à sources multiples suivant la revendication 1 ou 2, dans lequel la première (2) et la deuxième (4) sources de rayonnement X se distinguent, au moins, d'un facteur par la puissance de rayonnement X qu'ils peuvent produire au maximum pendant une durée de prise de vue déterminée.

4. Système (1) de tomographie par ordinateur à sources multiples suivant la revendication 3, dans lequel le facteur est plus grand que 1,3.

5. Système (1) de tomographie par ordinateur à sources multiples suivant l'une des revendications 1 à 4, dans lequel la première source (2) de rayons X a une anode tournante à montage magnétique.

6. Système (1) de tomographie par ordinateur à sources multiples suivant l'une des revendications 1 à 5, dans lequel il peut être produit, par la première source (2) de rayons X, une puissance de rayonnement X d'au moins 100 kW pendant une durée de fonctionnement d'au moins 2 s.

7. Système (1) de tomographie par ordinateur à sources multiples suivant l'une des revendications 1 à 6, dans lequel la deuxième source de rayons X est constituée sous la forme d'un tube à piston rotatif.

8. Système (1) de tomographie par ordinateur à sources multiples suivant l'une des revendications 1 à 7, dans lequel la deuxième source (4) de rayons X peut produire une puissance de rayons X d'au moins 60 kW pendant une durée de fonctionnement d'au moins 30 s.
